# FASCICULE DE BREVET EUROPEEN

(11) **EP 2 506 883 B1**
(45) Date de publication et mention de la délivrance du brevet: **25.09.2013**
(21) Numéro de dépôt: 10787749.0
(22) Date de dépôt: 03.12.2010
(51) Int. Cl.: A61L 2/08, B67C 3/26, B67C 7/00, B65B 55/08

(54) **SYSTEME DE PROTECTION POUR DISPOSITIF DE TRAITEMENT DE RECIPIENTS PAR FAISCEAU D'ELECTRONS**
SYSTEM FÜR DEN SCHUTZ EINER BEHÄLTERBEHANDLUNGSVORRICHTUNG MIT EINEM ELEKTRONENSTRAHL
SYSTEM FOR PROTECTING A CONTAINER TREATMENT DEVICE BY MEANS OF AN ELECTRON BEAM

(30) Priorité: 03.12.2009 FR 0958648
(43) Date de publication de la demande: 10.10.2012
(73) Titulaire: Hema, 29000 Quimper (FR)
(72) Inventeur: LEJEUNE, Philippe, F-29000 Quimper (FR); MACQUET, Philippe, F-29000 Quimper (FR)
(74) Mandataire: Hays, Bertrand
(86) Numéro de dépôt international: PCT/EP2010/068882
(87) Numéro de publication internationale: WO 2011/067393

(56) Documents cités:
- WO-A1-98/42385
- WO-A1-2005/002973
- US-A1- 2009 045 350
- US-B2- 6 668 877
- US-B2- 6 690 020

## Description

La présente invention concerne un dispositif de traitement pour la stérilisation de récipients par faisceau d'électrons, ledit dispositif étant équipé d'un système de protection particulier.

Il est connu, notamment par le document brevet US2009/0045350, des dispositifs de traitement comprenant un carrousel rotatif comportant un plateau support rotatif portant une pluralité de postes de traitement disposés à espace angulaire régulier, chaque poste de traitement comprenant des moyens de stérilisation qui comportent un émetteur de faisceau d'électrons, et des moyens de support pour supporter un récipient sous ledit émetteur, ledit émetteur étant apte à émettre un faisceau d'électron passant par l'ouverture supérieure d'un récipient porté par lesdits moyens de support, afin de stériliser le récipient, en particulier la paroi interne dudit récipient.

Par rapport à une stérilisation classique par voie chimique, la stérilisation par faisceau d'électrons se révèle plus efficace en termes de stérilisation, plus rapide, et ne laisse pas de traces résiduelles après traitement.

Les émetteurs de faisceaux d'électrons produisent toutefois des rayonnements indésirables, notamment des rayons X, et nécessitent donc de prévoir des systèmes de protection ou blindage pour éviter tout risque de propagation de rayonnements vers l'extérieur, et ainsi protéger les opérateurs. Les systèmes de protection sont formés par une enceinte de protection réalisée à base de plomb, dans laquelle est placé le carrousel rotatif pour la stérilisation, ainsi que les étoiles d'entrée et de sortie des récipients. De tels systèmes de protection s'avèrent lourds, encombrants, et très coûteux.

Le but de la présente invention est de proposer un dispositif de traitement pour la stérilisation de récipient avant remplissage, visant à pallier les inconvénients précités, qui soit performant et rapide, tout en restant simple de conception et de mise en oeuvre.

A cet effet, la présente invention propose un dispositif de traitement pour la stérilisation de récipients par faisceau d'électrons, comprenant
- un carrousel comportant un plateau support rotatif, monté rotatif sur un bâti fixe, portant une pluralité de postes de traitement disposés à espace angulaire régulier, chaque poste de traitement comprenant des moyens de stérilisation, qui comportent un émetteur de faisceau d'électrons, et des moyens de support pour supporter un récipient sous ledit émetteur, ledit émetteur étant apte à émettre un faisceau d'électrons passant par l'ouverture supérieure d'un récipient porté par lesdits moyens de support afin de stériliser ledit récipient, en particulier la paroi interne dudit récipient, et
- un système de protection pour stopper les rayonnements émis par les émetteurs,
caractérisé en ce que ledit système de protection comprend une goulotte fixe ouverte, sous la forme d'un segment annulaire, de préférence de section transversale en forme générale de U ou en V, montée fixe sous ledit plateau support et formant avec ce dernier une enceinte de protection en segment annulaire dans laquelle se déplacent les moyens de support portant les récipients,
ladite goulotte présentant une extrémité amont ouverte pour l'entrée des récipients à traiter dans l'enceinte de protection et une extrémité ouverte aval pour la sortie des récipients traités hors de l'enceinte.

Selon l'invention, la stérilisation est effectuée par faisceau d'électrons et le système de protection comprend une goulotte fixe formant avec un plateau support rotatif une enceinte de protection en segment annulaire dans laquelle les émetteurs seront activés pour effectuer les opérations de stérilisation. En comparaison des enceintes englobant l'ensemble du dispositif, l'enceinte de protection selon l'invention s'avère simple de conception et de réalisation, moins chère et moins encombrante.

Selon un mode de réalisation, ladite goulotte fixe est sensiblement sans contact avec ledit plateau support, ledit système de protection comprenant un premier système de chicanes pour faire écran aux rayonnements émis par les émetteurs à la liaison plateau-anneau, ledit premier système de chicanes comprenant par exemple deux nervures verticales s'étendant verticalement depuis la face inférieure du plateau, entre lesquelles la goulotte est positionnée.

Selon un mode de réalisation, ledit système de protection comprend un second système de chicanes pour faire écran aux rayonnements susceptibles de passer par les extrémités amont et aval de la goulotte.

Selon un mode de réalisation, le dispositif comprend en outre une première étoile de transfert, pour transférer les récipients à traiter vers les moyens de support en amont de l'extrémité amont, et une deuxième étoile de transfert pour évacuer les récipients traités des moyens de support en aval de l'extrémité aval. Ledit second système de chicane peut comprendre une paroi latérale sensiblement verticale s'étendant depuis la paroi extérieure de la goulotte, de part et d'autre des extrémités amont et aval, et entourant les deux étoiles de transfert.

Avantageusement, les éléments constitutifs du système de protection sont réalisés à base de plomb.

Selon une particularité, ledit émetteur est équipé d'une buse tubulaire, appelée également antenne, apte à s'étendre sensiblement verticalement sous le plateau support et à délivrer par son extrémité distale le faisceau d'électrons créé par l'émetteur, chaque poste de traitement comprenant des moyens de montée/descente, montés au dessus du plateau, agissant sur lesdits moyens support et/ou sur ledit émetteur pour introduire ladite buse dans le récipient, par son ouverture supérieure, ou pour extraire ladite buse du récipient. Selon un mode de réalisation, l'émetteur est monté fixe sur le plateau, sa buse s'étendant verticalement sous le plateau, les moyens de montée/descente étant aptes à déplacer lesdits moyens de support.

L'invention sera mieux comprise, et d'autres buts, détails, caractéristiques et avantages apparaîtront plus clairement au cours de la description explicative détaillée qui va suivre d'un mode de réalisation particulier actuellement préféré de l'invention, en référence aux dessins schématiques annexés, sur lesquels :
- la figure 1 est une vue schématique de dessus d'un dispositif de traitement selon l'invention, avec un arraché partiel du plateau support du carrousel ;
- la figure 2 est une vue schématique partielle en coupe radiale du carrousel au niveau d'un poste de traitement, la buse de l'émetteur étant disposée en dehors du récipient ;
- la figure 3 est une vue analogue à la figure 2, la buse de l'émetteur étant insérée dans le récipient ;
- la figure 4 est une vue schématique analogue à celle de la figure 2, illustrant une première variante de réalisation ; et,
- la figure 5 est une vue schématique analogue à celle de la figure 2, illustrant une deuxième variante de réalisation.

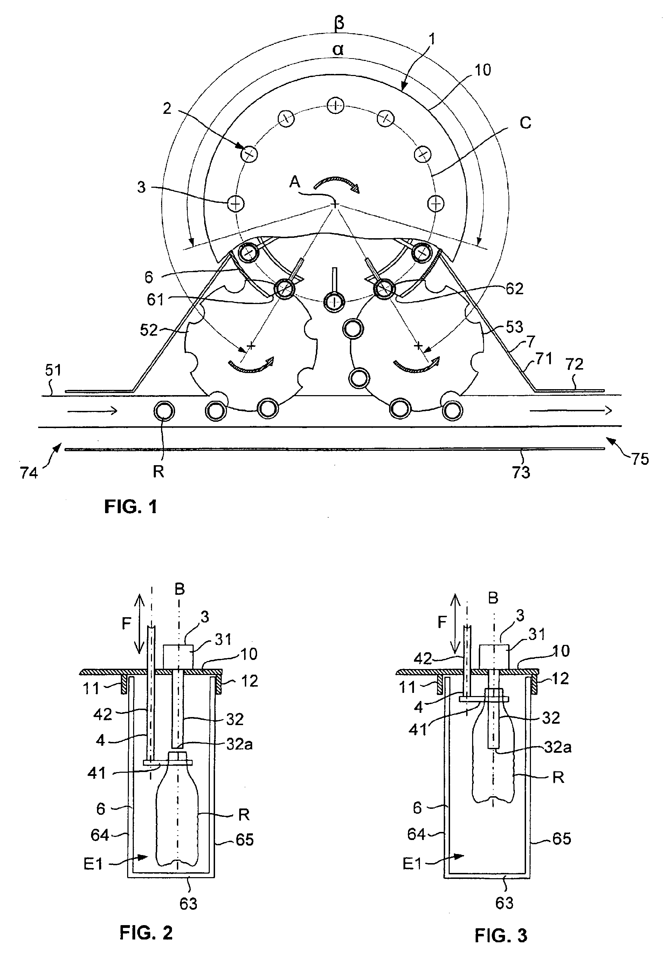

Les figures 1 à 3 illustrent un dispositif de traitement pour la stérilisation de récipients R, permettant de stériliser la paroi intérieure des récipients, en passant par l'ouverture supérieure des récipients, par exemple de bouteilles. Le dispositif pour la stérilisation est destiné à être placé en amont d'un dispositif pour le remplissage des récipients avec un produit de remplissage, par exemple un produit liquide tel que de l'eau, du lait ou du jus de fruit. Bien entendu, l'invention peut être appliquée à la stérilisation avant remplissage de tout type de récipient comprenant une ouverture principale supérieure, avec tout type de produit, liquide ou visqueux.

Le dispositif comprend un carrousel 1 comportant un plateau 10 support, sous la forme d'une plaque circulaire ou annulaire, destiné à être monté tournant sur un bâti fixe (non représenté) autour d'un axe A de rotation vertical, par exemple dans le sens horaire, tel qu'illustré sur la figure 1. Le plateau support porte une pluralité de postes 2 de traitement disposés à espace angulaire régulier autour de l'axe A de rotation.

Chaque poste de traitement comprend des moyens 3 de stérilisation formés d'un émetteur 31 de faisceau à électrons, connu en soi, muni d'une buse 32 tubulaire ou antenne tubulaire, de forme allongée l'émetteur étant apte à délivrer un faisceau d'électrons par l'extrémité 32a distale de sa buse. L'extrémité distale de la buse est équipée d'une feuille de titane, par exemple de 12µm d'épaisseur. Les moyens de stérilisation sont par exemple formés d'un émetteur tel que décrit dans le document brevet US 2008/0073549. L'émetteur est monté fixe sur la face supérieure du plateau. Sa buse, d'axe B longitudinal vertical, passe dans une ouverture du plateau 10 et s'étend sous la face inférieure du plateau. La buse est définie de sorte qu'elle puisse être insérée par l'ouverture supérieure des récipients pour irradier l'intérieur des récipients. Les buses sont disposées de sorte que leurs axes B soient disposés selon un cercle C.

Chaque poste de remplissage comprend en outre des moyens 4 de support d'un récipient R pour le maintien d'un récipient sous l'émetteur, centré selon l'axe B de la buse. Les moyens de support comprennent par exemple une pince 41 apte à saisir le récipient, par exemple au dessus ou en dessous de sa collerette située à la base de son col. La pince est montée sur une tige 42 verticale par laquelle les moyens de support sont montés sur le plateau 10. La tige est montée coulissante dans une ouverture du plateau et comprend une partie supérieure s'étendant au-dessus du plateau.

Cette partie supérieure de la tige est reliée à des moyens de montée/descente (non représentés) permettant de déplacer la pince en translation verticale, tel qu'illustré par la flèche F, entre une position basse illustrée à la figure 2, dans laquelle l'extrémité 32a distale de la buse 32 est disposée au-dessus du récipient, et une position haute dans laquelle la buse est insérée dans le récipient pour l'opération de stérilisation de la paroi interne du récipient.

Le dispositif comprend un système d'amenée de récipients, connu en soi, tel qu'un convoyeur 51 à bande combiné à un convoyeur tournant de transfert, classiquement appelé étoile 52 de transfert, qui permet d'engager latéralement les récipients entre les pinces des moyens de support des postes de traitement, et un système d'évacuation, comprenant une étoile 53 de transfert combinée par exemple à une portion aval du convoyeur 51 à bande précité, permettant d'évacuer latéralement les récipients stérilisés.

Le dispositif selon l'invention comprend un système de protection ou blindage pour stopper les rayonnements émis par les émetteurs, en particulier les rayonnements parasites du type rayons X.

Ce système de blindage comprend une goulotte 6 montée fixe sous le plateau 10, par exemple fixée au bâti du carrousel 1. Cette goulotte est ouverte et se présente donc sous la forme d'un segment annulaire s'étendant sur un secteur angulaire d'angle β, avec une extrémité 61 amont ouverte et une extrémité 62 aval ouverte. La goulotte présente une section transversale sensiblement en forme générale de U ou de V. Dans le mode de réalisation, la goulotte est sensiblement en forme de U et est formée d'une paroi 63 de fond, d'une paroi 64 cylindrique intérieure et d'une paroi 65 cylindrique extérieure. La goulotte est fixée de sorte que les bords libres de ses parois cylindriques soient disposés à proximité de la face inférieure du plateau, sans contact avec cette dernière, sa paroi de fond étant disposée sensiblement parallèle audit plateau. La goulotte est sensiblement centrée selon le cercle C décrit par les axes B des buses.

Le plateau 10 et la goulotte 6 sont réalisés à base de plomb, et forment ensemble une enceinte E1 de protection, sous la forme de segment annulaire s'étendant sur le secteur angulaire d'angle β, par exemple d'environ 300° dans le mode de réalisation illustré.

Le plateau est muni de deux nervures 11, 12 annulaires entre lesquelles sont positionnées les parois 64, 65 cylindriques de la goulotte, sensiblement sans contact, afin de former un système de chicanes évitant toute propagation de rayonnements au niveau de l'interface goulotte-plateau.

L'étoile 52 de transfert transfère les récipients vers les pinces, juste en amont de l'extrémité 61 amont, la pince étant en position basse. La phase de stérilisation est effectuée sur un secteur angulaire d'angle α qui est inférieur au secteur angulaire d'angle β. Pour chaque poste de traitement, l'émetteur peut être activé lorsque la pince est en position basse, afin d'irradier la paroi externe du récipient. La pince est ensuite déplacée progressivement vers sa position basse, puis remontée vers sa position haute afin d'irradier l'ensemble de la paroi interne du récipient, ledit récipient étant confiné dans l'enceinte E1 de protection. La longueur de la buse 32 et la hauteur de l'enceinte E1 pourront être adaptées, de sorte que l'extrémité 32a distale de la buse soit disposée à proximité du fond du récipient R dans la position haute de la pince 41. Les récipients traités sont ensuite récupérés par l'étoile de transfert, juste en aval de l'extrémité 62 aval de la goulotte.

Afin d'augmenter l'angle α sur lequel le traitement est effectué, tout en évitant les risques de propagation de rayonnements, le système de protection comprend un système de chicanes, réalisé également en plomb, pour faire écran aux rayonnements susceptibles de sortir par les extrémités 61, 62 ouvertes de la goulotte fixe. Ce système de chicanes, comprend une paroi 7 latérale sensiblement verticale s'étendant depuis la paroi 65 extérieure de la goulotte, de part et d'autre des extrémités de la goulotte, en entourant les deux étoiles 52, 53 de transfert, avec des passage 74, 75 pour l'entrée et la sortie des récipients. Ladite paroi latérale comprend deux premières portions 71 s'étendant depuis la paroi extérieure de la goulotte et se prolongeant chacune par une deuxième portion 72 parallèle au convoyeur 51, une troisième portion 73 est disposée du côté extérieur dudit convoyeur, parallèlement aux deuxièmes portions 72, les deuxièmes portions formant avec la troisième portion lesdits passages 74, 75 pour l'entrée et la sortie des récipients dans le dispositif. Selon un mode de réalisation, ce système de chicanes comprend une paroi supérieure horizontale et une paroi inférieure horizontale (non représentées), disposées respectivement au-dessus et en dessous des étoiles de transfert, et reliées à la paroi latérale.

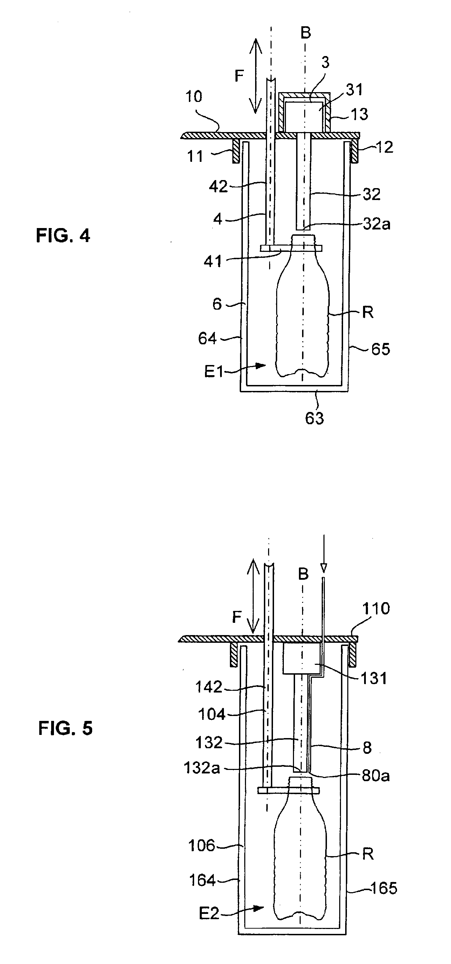

La figure 4 illustre un dispositif de traitement selon une variante de réalisation, qui se différencie du dispositif de traitement illustré aux figures 1 à 3 par le fait que les émetteurs 31 montés sur la face supérieure du plateau 10 sont recouverts d'un capot 13 afin de stopper les éventuels rayonnements émis directement par les émetteurs, en plus des rayonnements émis pas les buses des émetteurs. Chaque émetteur est recouvert d'un capot 13 de forme tubulaire, réalisé à base de plomb, avec une paroi périphérique tubulaire, par exemple de section transversale circulaire, fermée à son extrémité supérieure par une paroi supérieure. Le capot tubulaire est placé sur l'émetteur, avec son bord inférieur venant contre la face supérieure du plateau, et est assemblé au plateau par exemple de manière amovible. Le capot présente un passage (non représenté) pour la connexion électrique de l'émetteur. En variante, un seul capot annulaire recouvre l'ensemble des émetteurs. Le capot annulaire présente par exemple une section transversale sensiblement en forme générale de U, et est formé d'une paroi supérieure, d'une paroi cylindrique intérieure et d'une paroi cylindrique extérieure.

La figure 5 illustre un dispositif de traitement selon une deuxième variante de réalisation, dans laquelle les émetteurs 131 sont montés sur la face inférieure du plateau 110 du carrousel, de sorte que les émetteurs sont placés dans l'enceinte E2 de protection, formée comme précédemment par une goulotte 106 montée fixe sous le plateau 110. Pour permettre ce positionnement des émetteurs dans l'enceinte, le dispositif de traitement diffère de celui illustré aux figures 1 à 3 par le fait que la paroi 164 cylindrique intérieure et la paroi 165 cylindrique extérieure de la goulotte ont une hauteur plus importante, et que la tige 142 verticale des moyens 104 de support a une longueur plus importante.

La stérilisation par faisceau d'électrons peut générer une faible quantité d'ozone dans l'enceinte, en particulier dans le récipient placé dans l'enceinte. Selon un mode de réalisation, le dispositif de traitement comprend des moyens d'injection aptes à injecter un produit dans l'enceinte, et notamment dans le récipient, pendant et/ou après l'irradiation par faisceau d'électrons, pour chasser l'ozone généré lors de l'irradiation par les émetteurs de faisceau d'électrons, et ainsi éviter toute détérioration par l'ozone du produit de remplissage qui sera conditionné dans les récipients. Le produit injecté est un produit gazeux ou un produit liquide à bas point d'ébullition, notamment un gaz neutre tel que de l'azote, ou de l'azote liquide. Avantageusement, lesdits moyens d'injection comprennent une tubulure ou canule d'injection pour chaque poste de traitement, s'étendant de préférence le long de la buse de l'émetteur, ladite canule étant apte à s'introduire dans ledit récipient en passant par l'ouverture supérieure de ce dernier.

Tel qu'illustré à la figure 5, chaque poste de traitement comprend une canule 8 d'injection montée fixe sur le plateau 110, et qui traverse ce dernier de manière sensiblement étanche. Cette canule s'étend dans l'enceinte E2, sensiblement parallèlement à la buse 132 de l'émetteur, l'extrémité 80a distale de la canule étant disposée sensiblement au même niveau que l'extrémité 132a distale de la buse. La canule est utilisée pour injecter dans le récipient R un gaz neutre, tel que de l'azote, immédiatement après l'irradiation, afin de créer une surpression dans le récipient R et ainsi expulser hors du récipient l'ozone généré par l'irradiation. En variante, l'injection de gaz neutre est également réalisée lors de l'irradiation.

En variante, la canule est utilisée pour délivrer dans le récipient R de l'azote liquide, par exemple une goutte d'azote liquide, pendant et/ou juste après l'irradiation, l'azote liquide passant en phase gazeuse remplit alors progressivement le récipient et chasse progressivement l'ozone hors du récipient.

Les gaz contenus dans l'enceinte, tels qu'air, azote, ou ozone, sortent par les extrémités ouvertes de la goulotte. Avantageusement, le dispositif comprend un système de surpression d'air stérile, comportant des filtres qui stérilisent l'air et des pompes, pour assurer une surpression d'air stérile dans l'enceinte, afin de chasser notamment l'ozone de l'enceinte.

Bien que l'invention ait été décrite en liaison avec un mode de réalisation particulier, il est bien évident qu'elle n'y est nullement limitée et qu'elle comprend tous les équivalents techniques des moyens décrits ainsi que leurs combinaisons si celles-ci entrent dans le cadre de l'invention.

## Revendications

1. Dispositif de traitement pour la stérilisation de récipients (R) par faisceau d'électrons, comprenant
- un carrousel (1) comportant un plateau (10, 110) support rotatif, portant une pluralité de postes (2) de traitement disposés à espace angulaire régulier, chaque poste de traitement comprenant des moyens (3) de stérilisation qui comportent un émetteur (31, 131) de faisceau d'électrons et des moyens (4, 104) de support pour supporter un récipient sous ledit émetteur, ledit émetteur étant apte à émettre un faisceau d'électrons passant par l'ouverture supérieure d'un récipient porté par lesdits moyens de support afin de stériliser ledit récipient, et
- un système de protection pour stopper les rayonnements émis par les émetteurs,
**caractérisé en ce que** ledit système de protection comprend une goulotte (6, 106) fixe ouverte, sous la forme d'un segment annulaire, montée fixe sous ledit plateau (10, 110) support et formant avec ce dernier une enceinte (E1, E2) de protection dans laquelle se déplacent les moyens de support portant les récipients, ladite goulotte présentant une extrémité (61) amont ouverte pour l'entrée des récipients à traiter dans l'enceinte de protection et une extrémité (62) ouverte aval pour la sortie des récipients traités hors de l'enceinte.

2. Dispositif de traitement selon la revendication 1, **caractérisé en ce que** ladite goulotte (6, 106) fixe est sensiblement sans contact avec ledit plateau (10, 110) support, ledit système de protection comprenant un premier système (11, 12) de chicanes pour faire écran aux rayonnements émis par les émetteurs à la liaison plateau-anneau.

3. Dispositif de traitement selon la revendication 1 ou 2, **caractérisé en ce que** ledit système de protection comprend un second système (7) de chicanes pour faire écran aux rayonnements susceptibles de passer par les extrémités (61, 62) amont et aval de la goulotte (6, 106).

4. Dispositif de traitement selon l'une des revendications 1 à 3, **caractérisé en ce qu'**il comprend en outre une première étoile (52) de transfert, pour transférer les récipients (R) à traiter vers les moyens de support (4, 104) en amont de l'extrémité (61) amont, et une deuxième étoile (53) de transfert pour évacuer les récipients traités des moyens de support en aval de l'extrémité (62) aval.

5. Dispositif de traitement selon les revendications 3 et 4, **caractérisé en ce que** ledit second systèmes de chicane comprend une paroi (7) latérale sensiblement verticale s'étendant depuis la paroi (65) extérieure de la goulotte (6, 106), de part et d'autre des extrémités (61, 62) amont et aval, et entourant les deux étoiles (52, 53) de transfert.

6. Dispositif de traitement selon l'une des revendications 1 à 5, **caractérisé en ce que** les éléments (10, 11, 12, 13, 6, 7; 110, 106) constitutifs du système de protection sont réalisés à base de plomb.

7. Dispositif de traitement selon l'une des revendications 1 à 6, **caractérisé en ce que** ledit émetteur (31, 131) est équipé d'une buse (32, 132) tubulaire, apte à s'étendre sensiblement verticalement sous le plateau (10, 110) support et à délivrer par son extrémité (32a, 132a) distale le faisceau d'électrons créé par l'émetteur, chaque poste (2) de traitement comprenant des moyens de montée/descente, agissant sur lesdits moyens (4, 104) de support et/ou sur ledit émetteur (31, 131) pour introduire ladite buse dans le récipient, par son ouverture supérieure, ou pour extraire ladite buse du récipient.

8. Dispositif de traitement selon la revendication 7, **caractérisé en ce que** l'émetteur (31, 131) de chaque poste de traitement est monté fixe sur le plateau (10), sa buse s'étendant verticalement sous le plateau, les moyens de montée/descente étant aptes à déplacer lesdits moyens (4, 104) de support.

9. Dispositif de traitement selon la revendication 7, **caractérisé en ce que** l'émetteur (31) de chaque poste de traitement est monté fixe sur la face supérieure du plateau (10), ledit système de protection comprenant en outre un ou plusieurs capots (13) recouvrant lesdits émetteurs (31).

10. Dispositif de traitement selon la revendication 7, **caractérisé en ce que** l'émetteur (131) de chaque poste de traitement est monté fixe sur la face inférieure du plateau (110) et est disposé dans ladite enceinte (E2) de protection.

11. Dispositif de traitement selon l'une des revendications 1 à 10, **caractérisé en ce qu'**il comprend des moyens (8) d'injection aptes à injecter un produit dans l'enceinte (E2) de protection pour chasser l'ozone généré lors de l'irradiation par les émetteurs (31, 131) de faisceau d'électrons.

12. Dispositif de traitement selon la revendication 11, **caractérisé en ce que** lesdits moyens d'injection comprennent une canule (8) d'injection pour chaque poste de traitement, ladite canule étant apte à s'introduire dans ledit récipient (R) en passant par l'ouverture supérieure de ce dernier.

## Patentansprüche

1. Behandlungsvorrichtung zur Sterilisierung von Behältern (R) durch Elektronenstrahl, die enthält
- einen Drehständer (1), der eine drehbare Tragplatte (10, 110) aufweist, die eine Vielzahl von Behandlungsstationen (2) trägt, welche in gleichmäßigem Winkelabstand angeordnet sind, wobei jede Behandlungsstation Sterilisierungseinrichtungen (3) enthält, die einen Elektronenstrahlemitter (31, 131) und Trageinrichtungen (4, 104) aufweisen, um einen Behälter unter dem Emitter zu tragen, wobei der Emitter einen Elektronenstrahl emittieren kann, der durch die obere Öffnung eines von den Trageinrichtungen getragenen Behälters geht, um den Behälter zu sterilisieren, und
- ein Schutzsystem, um die von den Emittern emittierten Strahlungen zu stoppen,
**dadurch gekennzeichnet, dass** das Schutzsystem eine offene ortsfeste Rinne (6, 106) in Form eines Ringsegments enthält, die ortsfest unter der Tragplatte (10, 110) montiert ist und mit dieser einen Schutzraum (E1, E2) formt, in dem sich die die Behälter tragenden Trageinrichtungen verschieben, wobei die Rinne ein offenes vorderes Ende (61) für den Eintritt der zu behandelnden Behälter in den Schutzraum und ein hinteres offenes Ende (62) für den Austritt der behandelten Behälter aus dem Raum aufweist.

2. Behandlungsvorrichtung nach Anspruch 1, **dadurch gekennzeichnet, dass** die ortsfeste Rinne (6, 106) im Wesentlichen ohne Kontakt mit der Tragplatte (10, 110) ist, wobei das Schutzsystem ein erstes System (11, 12) von Blenden enthält, um an der Verbindung von Platte und Ring eine Abschirmung gegenüber den von den Emittern emittierten Strahlungen zu bilden.

3. Behandlungsvorrichtung nach Anspruch 1 oder 2, **dadurch gekennzeichnet, dass** das Schutzsystem ein zweites System (7) von Blenden enthält, um eine Abschirmung gegenüber den Strahlungen zu bilden, die durch die vorderen und hinteren Enden (61, 62) der Rinne (6, 106) gehen können.

4. Behandlungsvorrichtung nach einem der Ansprüche 1 bis 3, **dadurch gekennzeichnet, dass** es außerdem einen ersten Übertragungsstern (52), um die zu behandelnden Behälter (R) zu den Trageinrichtungen (4, 104) vor dem vorderen Ende (61) zu übertragen, und einen zweiten Übertragungsstern (53) enthält, um die behandelten Behälter von den Trageinrichtungen hinter dem hinteren Ende (62) zu entfernen.

5. Behandlungsvorrichtung nach den Ansprüchen 3 und 4, **dadurch gekennzeichnet, dass** das zweite Blendensystem eine im Wesentlichen senkrechte Seitenwand (7) enthält, die sich von der Außenwand (65) der Rinne (6, 106) zu beiden Seiten der vorderen und hinteren Enden (61, 62) erstreckt und die zwei Übertragungssterne (52, 53) umgibt.

6. Behandlungsvorrichtung nach einem der Ansprüche 1 bis 5, **dadurch gekennzeichnet, dass** die das Schutzsystem bildenden Elemente (10, 11, 12, 13, 6, 7; 110, 106) auf der Basis von Blei hergestellt werden.

7. Behandlungsvorrichtung nach einem der Ansprüche 1 bis 6, **dadurch gekennzeichnet, dass** der Emitter (31, 131) mit einer rohrförmigen Düse (32, 132) ausgestattet ist, die sich im Wesentlichen senkrecht unter der Tragplatte (10, 110) erstrecken und über ihr distales Ende (32a, 132a) den vom Emitter erzeugten Elektronenstrahl liefern kann, wobei jede Behandlungsstation (2) Aufstiegs-/Abstiegseinrichtungen enthält, die auf die Trageinrichtungen (4, 104) und/oder den Emitter (31, 131) einwirken, um die Düse in den Behälter über seine obere Öffnung einzuführen, oder um die Düse aus dem Behälter zu entnehmen.

8. Behandlungsvorrichtung nach Anspruch 7, **dadurch gekennzeichnet, dass** der Emitter (31, 131) jeder Behandlungsstation ortsfest auf die Platte (10) montiert ist, wobei seine Düse sich senkrecht unter der Platte erstreckt, wobei die Aufstiegs-/Abstiegseinrichtungen die Trageinrichtungen (4, 104) verschieben können.

9. Behandlungsvorrichtung nach Anspruch 7, **dadurch gekennzeichnet, dass** der Emitter (31) jeder Behandlungsstation ortsfest auf die Oberseite der Platte (10) montiert ist, wobei das Schutzsystem außerdem eine oder mehrere die Emitter (31) bedeckende Kappen (13) enthält.

10. Behandlungsvorrichtung nach Anspruch 7, **dadurch gekennzeichnet, dass** der Emitter (131) jeder Behandlungsstation ortsfest auf die Unterseite der Platte (110) montiert und im Schutzraum (E2) angeordnet ist.

11. Behandlungsvorrichtung nach einem der Ansprüche 1 bis 10, **dadurch gekennzeichnet, dass** sie Einspritzeinrichtungen (8) enthält, die ein Produkt in den Schutzraum (E2) spritzen können, um das bei der Bestrahlung durch die Elektronenstrahlemitter (31, 131) erzeugte Ozon zu verdrängen.

12. Behandlungsvorrichtung nach Anspruch 11, **dadurch gekennzeichnet, dass** die Einspritzeinrichtungen eine Einspritzkanüle (8) für jede Behandlungsstation enthalten, wobei die Kanüle über dessen obere Öffnung in den Behälter (R) eindringen kann.

## Claims

1. Treatment device for sterilising containers (R) by means of an electron beam, comprising
- a carousel (1) comprising a rotary holding plate (10, 110), supporting a plurality of treatment stations (2) arranged with regular angular spacing, each treatment station comprising sterilisation means (3) which include an electron beam emitter (31, 131) and holding means (4, 104) to hold a container under said emitter, said emitter being able to emit an electron beam passing through the upper opening of a container supported by said holding means in order to sterilise said container, and
- a protection system for stopping the rays emitted by the emitters,
**characterised in that** said protection system comprises an open stationary channel (6, 106), in the form of a ring-shaped segment, mounted in a stationary manner under said holding plate (10, 110) and forming with the latter a protective chamber (E1, E2) wherein the holding means supporting the containers moves, said channel having an open upstream end (61) for the inlet of the containers to be treated into the protective chamber and an open downstream end (62) for the outlet of the treated containers from the chamber.

2. Treatment device according to claim 1, **characterised in that** said stationary channel (6, 106) is substantially without contact with said holding plate (10, 110), said protection system comprising a first system (11, 12) of baffles to shield against the rays emitted by the emitters at the plate-ring liaison.

3. Treatment device according to claim 1 or 2, **characterised in that** said protection system comprises a second system (7) of baffles to shield against the rays that can pass through the upstream and downstream ends (61, 62) of the channel (6, 106).

4. Treatment device according to one of claims 1 to 3, **characterised in that** it further comprises a first transfer starwheel (52), to transfer the containers (R) to be treated to the holding means (4, 104) upstream of the upstream end (61), and a second transfer starwheel (53) to remove the treated containers from the holding means downstream of the downstream end (62).

5. Treatment device according to claims 3 and 4, **characterised in that** said second system of baffles comprises a substantially vertical lateral wall (7) extending from the outer wall (65) of the channel (6, 106), on either side of the upstream and downstream ends (61, 62), and surrounding the two transfer starwheels (52, 53).

6. Treatment device according to one of claims 1 to 5, **characterised in that** the elements (10, 11, 12, 13, 6, 7; 110, 106) that comprise the protection system are made from a lead base.

7. Treatment device according to one of claims 1 to 6, **characterised in that** said emitter (31, 131) is provided with a tubular nozzle (32, 132), able to extend substantially vertically under the holding plate (10, 110) and to deliver via its distal end (32a, 132a) the electron beam created by the emitter, each treatment station (2) comprising means of raising/lowering, acting on said holding means (4, 104) and/or on said emitter (31, 131) in order to introduce said nozzle in the container, via its upper opening, or in order to extract said nozzle from the container.

8. Treatment device according to claim 7, **characterised in that** the emitter (31, 131) of each treatment station is mounted in a stationary manner on the plate (10), its nozzle extending vertically under the plate, the means of raising/lowering being able to move said holding means (4, 104).

9. Treatment device according to claim 7, **characterised in that** the emitter (31) of each treatment station is mounted in a stationary manner on the upper face of the plate (10), said protection system further comprising one or several covers (13) covering said emitters (31).

10. Treatment device according to claim 7, **characterised in that** the emitter (131) of each treatment station is mounted in a stationary manner on the lower face of the plate (110) and is arranged in said protective chamber (E2).

11. Treatment device according to one of claims 1 to 10, **characterised in that** it comprises means of injecting (8) able to inject a product into the protective chamber (E2) in order to remove the ozone generated during the irradiation by the electron beam emitters (31, 131).

12. Treatment device according to claim 11, **characterised in that** said means of injection include an injection cock (8) for each treatment station, said cock being able to be introduced into said container (R) by passing through the upper opening of the latter.
